# EUROPEAN PATENT APPLICATION

(11) **EP 4 502 586 A1**
(43) Date of publication of application: **05.02.2025**
(21) Application number: 23779675.0
(22) Date of filing: 16.03.2023
(51) Int. Cl.: G01N 27/22, G01N 27/04, G01N 27/06

(54) **FLUID PROPERTIES MONITORING SYSTEM, METHOD FOR DETECTING ABNORMALITY IN VALUES DETECTED BY FLUID PROPERTIES SENSOR, AND PROGRAM**

(30) Priority: 31.03.2022 JP 2022059441
(71) Applicant: KYB Corporation, Minato-ku, Tokyo 105-5128 (JP)
(72) Inventor: YOSHIDA, Takahiro, Tokyo 105-5128 (JP); KAMEDA, Yukinori, Tokyo 105-5128 (JP)
(74) Representative: Grünecker Patent- und Rechtsanwälte PartG mbB
(86) International application number: PCT/JP2023/010316
(87) International publication number: WO 2023/189657

(57) **Abstract**

A fluid property monitoring system (100) includes an information processing unit (30) to which a detected value from a fluid property sensor (10) and maintenance information are input, the fluid property sensor (10) being attached to a fluid pressure apparatus (1) and being configured to detect a property of fluid, and the maintenance information being input by an operator and including a state of the fluid pressure apparatus (1), the information processing unit (30) is configured to detect an abnormality in the detected value by comparing the detected value and an abnormality determination value set based on the maintenance information.

## Description

### TECHNICAL FIELD

The present invention relates to a fluid property monitoring system, a method for detecting an abnormality in a detected value from a fluid property sensor, and a program.

### BACKGROUND ART

JP2019-49495A discloses a fluid property detection device for detecting a property of a fluid to be detected. The fluid property detection device is attached directly to a fluid pressure apparatus, such as a hydraulic cylinder, etc., that is driven by utilizing working oil as working fluid or is attached to a piping that is connected to the hydraulic cylinder, etc., thereby detecting a property of the working oil.

### SUMMARY OF INVENTION

The fluid property detection device as described in JP2019-49495A does not have a mechanism for detecting a failure of the fluid property detection device itself. Therefore, there is a risk in that the failure of the fluid property detection device cannot be noticed, and a detection accuracy of the property of the working oil is deteriorated.

An object of the present invention is to prevent deterioration of a detection accuracy of a property of a fluid due to an abnormality of a fluid property sensor.

According to one aspect of the present invention, a fluid property monitoring system includes an information processing unit to which a detected value from a fluid property sensor and maintenance information are input, the fluid property sensor being attached to a fluid pressure apparatus and being configured to detect a property of fluid, and the maintenance information being input by an operator and including a state of the fluid pressure apparatus, the information processing unit is configured to detect an abnormality in the detected value by comparing the detected value and an abnormality determination value set based on the maintenance information.

According to other aspect of the present invention, a method for detecting an abnormality in a detected value from a fluid property sensor that is attached to a fluid pressure apparatus and detects a property of a fluid, the abnormality in the detected value is detected by comparing the detected value from the fluid property sensor with an abnormality determination value, the abnormality determination value being set based on maintenance information including a state of the fluid pressure apparatus, and the maintenance information being input by an operator.

According to other aspect of the present invention, a program for causing a computer to execute detection of an abnormality in a detected value from a fluid property sensor that is attached to fluid pressure apparatus and detects a property of a fluid, the abnormality in the detected value is detected by comparing the detected value from the fluid property sensor with an abnormality determination value, the abnormality determination value being set based on maintenance information including a state of the fluid pressure apparatus, and the maintenance information being input by an operator.

### BRIEF DESCRIPTION OF DRAWINGS

[FIG. 1] FIG. 1 is a schematic view of a fluid property monitoring system according to an embodiment of the present invention.
[FIG. 2] FIG. 2 is a diagram showing a relationship between elapsed time during operation of a fluid pressure apparatus and an electric characteristic value of working fluid.
[FIG. 3] FIG. 3 is a diagram showing changes in the electric characteristic value due to maintenance of the fluid pressure apparatus.
[FIG. 4] FIG. 4 is a flowchart showing a processing procedure for an information processing unit to output a maintenance signal.
[FIG. 5] FIG. 5 is a flowchart showing a processing procedure for the information processing unit to output an abnormality signal.
[FIG. 6] FIG. 6 is a flowchart showing a processing procedure for the information processing unit to output a warning signal.
[FIG. 7] FIG. 7 is a diagram showing changes in the electric characteristic value when it is considered that the maintenance of the fluid pressure apparatus has been performed.

### DESCRIPTION OF EMBODIMENTS

A fluid property monitoring system 100 according to an embodiment of the present invention will be described with reference to the drawings.

The fluid property monitoring system 100 is used for a fluid pressure apparatus 1, such as a hydraulic pump, a hydraulic motor, a hydraulic valve, a hydraulic cylinder, and so forth, that is driven by utilizing working oil as working fluid, for example. The fluid property monitoring system 100 detects and monitors a property of the working fluid, such as the working oil, working water, compressed air, and so forth, that is to be supplied/ discharged to/from the fluid pressure apparatus 1 and notifies an operator of degradation of the working fluid and an abnormality of the fluid property monitoring system 100 itself. Then, the operator replaces or adds the working fluid of the fluid pressure apparatus 1 and/or performs maintenance of the fluid property monitoring system 100 itself, for example. A monitoring target of the fluid property monitoring system 100 is not limited to the working fluid of the fluid pressure apparatus 1, and may be various liquids or gases such as lubricating oils, cutting oils, fuels, solvents, and chemicals. Hereinafter, the working fluid of the fluid pressure apparatus 1 that is the monitoring target of the fluid property monitoring system 100 may simply be referred to as "the working fluid".

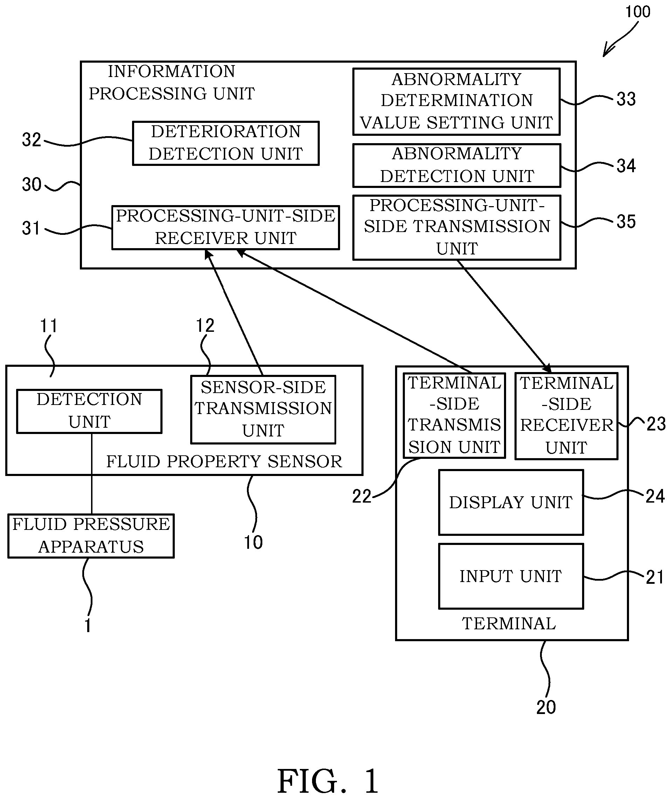

As shown in FIG. 1, the fluid property monitoring system 100 includes: a fluid property sensor 10 that is attached to the fluid pressure apparatus 1 and detects a property of a fluid; a terminal 20 to which maintenance information including a state of the fluid pressure apparatus 1 is input by the operator; and an information processing unit 30 to which a detected value and the maintenance information of the fluid property sensor 10 are input.

The fluid property sensor 10 is attached, for example, directly to the fluid pressure apparatus 1, or to a piping connected to the fluid pressure apparatus 1, and the fluid property sensor 10 detects the property of the working fluid supplied/discharged to/from the fluid pressure apparatus 1. Specifically, the fluid property sensor 10 detects electric characteristic values such as the dielectric constant and conductivity, temperature, and so forth of the working fluid in order to monitor deterioration of the working fluid.

The fluid property sensor 10 has a detection unit 11 that detects the property of the working fluid and a sensor-side transmission unit 12 that sends the detection result from the detection unit 11 to the information processing unit 30. In this embodiment, the detection unit 11 has a pair of electrodes (not shown) that are provided so as to oppose with each other. The detection unit 11 detects the electric characteristic values, such as the dielectric constant, the conductivity, and so forth, of the working fluid from a value of voltage applied to the pair of electrodes and a value of current flowing between the pair of electrodes. In addition, the detection unit 11 detects temperature with a temperature sensor that is attached to one of the electrodes. Because it is possible to employ a known structure for the detection unit 11, detailed illustration and description of the configuration are omitted. In addition, in the following, although a description will be given of a case in which the electric characteristic values to by detected by the fluid property sensor 10 are the dielectric constant and the conductivity, the electric characteristic values to be detected by the fluid property sensor 10 are not limited to the dielectric constant and the conductivity. The sensor-side transmission unit 12 transmits and outputs the detected value from the detection unit 11 to the information processing unit 30 continuously or at regular intervals via wireless communication.

The terminal 20 is, for example, a portable terminal, such as a smartphone, or a personal computer. The terminal 20 outputs the maintenance information input by the operator to the information processing unit 30, and as described later, receives, from the information processing unit 30, a maintenance signal encouraging maintenance of the fluid pressure apparatus 1, an abnormality signal notifying an abnormality of the fluid property sensor 10, and a warning signal warning the operator. Thereby, the operator can understand through the terminal 20 whether or not the maintenance of the fluid pressure apparatus 1 is required, whether or not there is an abnormality of the fluid property sensor 10, and so forth.

The terminal 20 has an input unit 21 that accepts input of the maintenance information including the state of the fluid pressure apparatus 1 by the operator, a terminal-side transmission unit 22 that transmits the maintenance information that has input to the input unit 21 to the information processing unit 30, a terminal-side receiver unit 23 that receives information from the information processing unit 30, and a display unit 24 that displays various information.

The input unit 21 is, for example, a touch panel of a smartphone, a keyboard of a personal computer, and so forth. An application for assisting the input of the maintenance information is installed in the terminal 20, and the operator inputs the maintenance information of the fluid pressure apparatus 1 to the input unit 21 in accordance with the display of the application. Specifically, the maintenance information to be input to the input unit 21 includes: the date and time of replacement or addition of the working fluid, the date and time of replacement of a filter installed in the fluid pressure apparatus 1 for removing foreign matter from the working fluid, the date and time when the abnormality was detected in the fluid pressure apparatus 1 and the content of the abnormality, the state of the fluid pressure apparatus 1, and so forth. The "state of the fluid pressure apparatus 1" refers to information from which the operator can estimate the degree of degradation, etc. of the working fluid, such as an increase in the operating sound of the fluid pressure apparatus 1, for example, and is routine maintenance information of the fluid pressure apparatus 1 that can be obtained without performing any modification to the fluid pressure apparatus 1. As described above, the maintenance information includes the information that can be obtained by actually performing the modification of the fluid pressure apparatus 1 and the information that can be obtained without performing the modification of the fluid pressure apparatus 1. The maintenance information to be input to the input unit 21 is not limited to the above as long as it is information from which the degree of degradation of the working fluid can be estimated.

The terminal-side transmission unit 22 transmits the maintenance information that has been input to the input unit 21 to the information processing unit 30 via the wireless communication. In addition, as described later, the terminal-side receiver unit 23 receives the maintenance signal, the abnormality signal, and the warning signal that have been transmitted from the information processing unit 30 via the wireless communication, and on the basis of the received signals, the display unit 24 displays the various information. Specifically, when the terminal-side receiver unit 23 receives the maintenance signal, the display unit 24 displays the information encouraging the maintenance of the fluid pressure apparatus 1. In addition, when the terminal-side receiver unit 23 receives the abnormality signal, the display unit 24 displays the information encouraging the maintenance of the fluid property sensor 10. In addition, when the terminal-side receiver unit 23 receives the warning signal, the display unit 24 displays the information encouraging input of the maintenance information and displays that there is a possibility of abnormality of the fluid property sensor 10.

The information processing unit 30 has a processing unit such as a CPU, etc., a storage device, a display device, an input device, a communication device, and so forth, and the CPU executes a program that is stored in the storage device in advance to execute each of processes performed by the information processing unit 30, which will be described later. In this embodiment, the information processing unit 30 is a server on the cloud that communicates with the fluid property sensor 10 and the terminal 20 via the wireless communication. The information processing unit 30 is not limited to a server on the cloud, and may be a device (a computer) that communicates with the fluid property sensor 10 and the terminal 20 via the wireless communication or wired communication. As described above, the information processing unit 30 communicates with the fluid property sensor 10 and the terminal 20 via a network. The information processing unit 30 determines whether or not the maintenance of the fluid pressure apparatus 1 is required from the detected value input from the fluid property sensor 10, and outputs the maintenance signal to the terminal 20 when it is determined that the maintenance is required. In addition, from the detected value that is input from the fluid property sensor 10 and the maintenance signal that is output from the terminal 20, the information processing unit 30 detects the abnormality in the detected value from the fluid property sensor 10 and outputs it to the terminal 20 as the abnormality signal, and detects the possibility of the abnormality of the fluid property sensor 10 and outputs it to the terminal 20 as the warning signal.

The information processing unit 30 has: a processing-unit-side receiver unit 31 that receives the detected value from the fluid property sensor 10 that is transmitted from the sensor-side transmission unit 12 of the fluid property sensor 10 and the maintenance information that is transmitted from the terminal-side transmission unit 22 of the terminal 20; a deterioration detection unit 32 that detects the deterioration of the working fluid from the detected value from the fluid property sensor 10; an abnormality determination value setting unit 33 that sets an abnormality determination value on the basis of the maintenance information that is input from the terminal 20; an abnormality detection unit 34 that detects the abnormality in the detected value from the fluid property sensor 10 by comparing the abnormality determination value and the detected value from the fluid property sensor 10; and a processing-unit-side transmission unit 35 that performs the notification by transmitting the detection results from the deterioration detection unit 32 and the abnormality detection unit 34 to the terminal 20. The processing-unit-side receiver unit 31, etc. are shown as virtual units for the respective functions of the information processing unit 30, and they do not imply that they physically exist.

Through the processing-unit-side receiver unit 31, the detected value is input from the fluid property sensor 10. In addition, through the processing-unit-side receiver unit 31, the maintenance information of the fluid pressure apparatus 1 is input from the terminal 20.

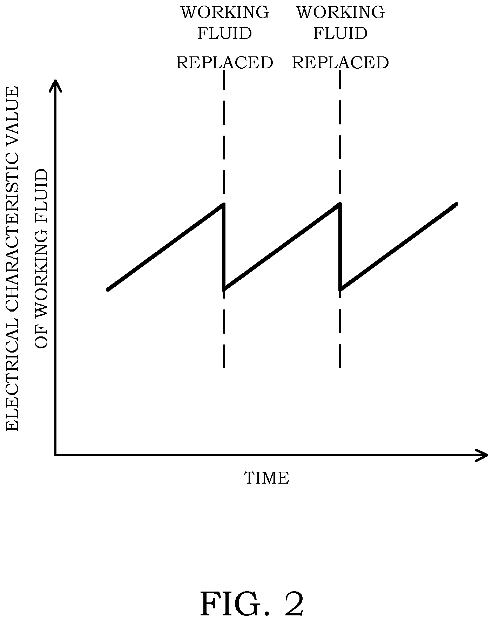

From the detected value from the fluid property sensor 10, the deterioration detection unit 32 detects the deterioration of the working fluid of the fluid pressure apparatus 1. Specifically, the deterioration detection unit 32 determines whether or not the maintenance, such as the replacement of the working fluid, etc., is required from the electric characteristic value of the working fluid detected by the fluid property sensor 10. In the following, a case in which the replacement of the working fluid is performed as the maintenance will be described. In this embodiment, as shown in FIG. 2, the electric characteristic value of the working fluid (the dielectric constant or the conductivity) is increased with the deterioration of the working fluid. The deterioration detection unit 32 determines whether or not the electric characteristic value of the working fluid detected by the fluid property sensor 10 falls within a predetermined threshold value (whether or not the electric characteristic value is equal to or less than the predetermined threshold value). If the electric characteristic value does not fall within the threshold value (if the electric characteristic value is larger than the threshold value), it is determined that the working fluid is deteriorated and the replacement thereof is required, and the maintenance signal is output to the terminal 20 via the processing-unit-side transmission unit 35. Note that the threshold value for determining whether or not the maintenance is required is not limited to a single value, and may be a numerical range having a certain numerical width. In addition, even when the electric characteristic value falls within the threshold value and it is determined that the maintenance is not required, a signal for notifying the operator of the degree of degradation of the working fluid may be output.

As described above, in a state in which the deterioration of the working fluid is detected, it is not only possible to consider that the working fluid is actually deteriorated, but also possible to consider the possibility that the working fluid is not deteriorated and that the deterioration of the working fluid is erroneously detected due to the abnormality of the fluid property sensor 10. Therefore, in the fluid property monitoring system 100, the abnormality in the detected value from the fluid property sensor 10 is detected by the abnormality determination value setting unit 33 and the abnormality detection unit 34, and it is notified to the operator.

The abnormality determination value setting unit 33 sets an abnormality detection value for detecting the abnormality in the detected value from the fluid property sensor 10 by estimating the detected value from the fluid property sensor 10 from the maintenance information. In this embodiment, as shown in FIG. 2, the electric characteristic value of the working fluid (the dielectric constant, the conductivity) to be detected by the fluid property sensor 10 is increased with the deterioration of the working fluid due to an increase in the operation time of the fluid pressure apparatus 1, and the electric characteristic value is decreased as the working fluid is replaced with a fresh working fluid which is not deteriorated. In the information processing unit 30, an electric characteristic value map indicating the correlation between the elapsed time since the working fluid was replaced and the electric characteristic value of the working fluid is stored in advance. In FIG. 2, the electric characteristic value map indicates the correlation between the electric characteristic value of the working fluid and the elapsed time during the period between a replacement of the working fluid and next replacement of the working fluid. The electric characteristic value map is not limited to a map stored in advance in the information processing unit 30, and a map in which the detection results from the fluid property sensor 10 are accumulated may be used.

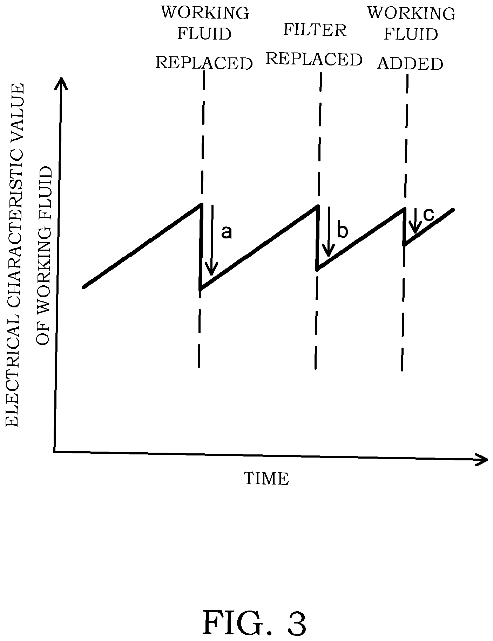

On the basis of the electric characteristic value map, the abnormality determination value setting unit 33 sets the abnormality detection value that is the threshold value of the electric characteristic value that the working fluid can take. Specifically, if it is immediately after the replacement of the working fluid, the abnormality determination value setting unit 33 sets, as the abnormality detection value, a value that considers the variation of the electric characteristic value for the electric characteristic value of the working fluid immediately after the replacement in the electric characteristic value map. In addition, if some time has passed since the replacement of the working fluid, the abnormality determination value setting unit 33 sets the abnormality detection value on the basis of the elapsed time and the electric characteristic value map. As shown in FIG. 3, when fresh working fluid is added or the filter of the fluid pressure apparatus 1 is replaced as the maintenance, a change in the electric characteristic value of the working fluid (b and c shown in FIG. 3) is smaller than that for the replacement of the working fluid (a shown in FIG. 3). Therefore, if it is immediately after the addition of the working fluid or immediately after the replacement of the filter of the fluid pressure apparatus 1, the abnormality determination value setting unit 33 sets, as the abnormality detection value, the threshold value that is larger than that immediately after the replacement of the working fluid. The abnormality detection value set by the abnormality determination value setting unit 33 is output to the abnormality detection unit 34.

The abnormality determination value setting unit 33 may set the abnormality detection value using not only the electric characteristic value map, but also using calculation. Specifically, the abnormality determination value setting unit 33 may calculate the changed value (a decreased value) of the electric characteristic value due to the replacement of the working fluid and calculate the changed value (an increased value) of the electric characteristic value caused over the time elapsed since the replacement of the working fluid, thereby setting the abnormality detection value. In the following, a case in which the threshold value of a single value is set as the abnormality detection value will be described.

The abnormality detection unit 34 determines whether or not the detected value from the fluid property sensor 10 falls within the threshold value of the abnormality detection value (specifically, equal to or less than the threshold value). If the detected value from the fluid property sensor 10 falls within the threshold value of the abnormality detection value, the abnormality detection unit 34 does not detect the abnormality of the fluid property sensor 10. On the other hand, if the detected value from the fluid property sensor 10 does not fall within the threshold value of the abnormality detection value, the abnormality detection unit 34 detects the abnormality in the detected value from the fluid property sensor 10 and outputs the abnormality signal notifying the abnormality of the fluid property sensor 10 to the terminal 20 via the processing-unit-side transmission unit 35.

As shown in FIG. 2, it is expected that the electric characteristic value to be detected by the fluid property sensor 10 will be decreased immediately after the replacement of the working fluid. However, if this change is not reflected to the electric characteristic value detected by the fluid property sensor 10 and the electric characteristic value remains high, an abnormality of the fluid property sensor 10 is suspected. Thus, in a case in which the electric characteristic value detected by the fluid property sensor 10 remains high and is not decreased despite immediately after the replacement of the working fluid, the abnormality detection unit 34 detects the abnormality in the detected value from the fluid property sensor 10.

In addition, during the operation of the fluid pressure apparatus 1, it is expected that the electric characteristic value to be detected by the fluid property sensor 10 will be increased until the replacement of the working fluid. However, in a case in which this change is not reflected to the electric characteristic value detected by the fluid property sensor 10 and the electric characteristic value is decreased or is not increased, the abnormality of the fluid property sensor 10 is suspected. Thus, the abnormality detection unit 34 detects the abnormality in the detected value from the fluid property sensor 10 in a case in which the electric characteristic value detected by the fluid property sensor 10 is decreased even when the working fluid is not replaced, or in a case in which the electric characteristic value is not increased even when some time has passed since the replacement of the working fluid.

As described above, a method for detecting the abnormality in the detected value from the fluid property sensor 10 detects an abnormality in the detected value by comparing the detected value from the fluid property sensor 10 with the abnormality determination value that is set on the basis of the maintenance information including the state of the fluid pressure apparatus 1 that is input by the operator. In the fluid property monitoring system 100, the abnormality in the detected value from the fluid property sensor 10 is detected by comparing the detected value from the fluid property sensor 10 that is estimated from the maintenance information with the actual detected value from the fluid property sensor 10. In other words, in the fluid property monitoring system 100, it is possible to determine whether the cause of the detection of the deterioration of the working fluid is due to the actual deterioration of the working fluid or due to the abnormality of the fluid property sensor 10. As a result, it is possible to encourage the operator to perform the maintenance of the fluid property sensor 10, and thus, a decrease in the detection accuracy of the property of the fluid due to the abnormality of the fluid property sensor 10 is prevented.

In addition, in a state in which the abnormality in the detected value from the fluid property sensor 10 is detected, it is not only possible to consider that the abnormality is actually caused in the fluid property sensor 10, but also possible to consider the possibility that there is no abnormality in the fluid property sensor 10 and the maintenance information related to the replacement of the working fluid, etc. performed by the operator has not been input. In a case in which the maintenance information has not been input, the change in the electric characteristic value is caused by the maintenance, such as the replacement of the working fluid, etc., but not by the abnormality of the fluid property sensor 10, and there is a possibility that the fluid property sensor 10 is normal. Thus, the abnormality detection unit 34 outputs the warning signal when the detected value from the fluid property sensor 10 is changed in a similar manner to a case in which the maintenance has been performed, but the operator has not input the maintenance information. The warning signal is a signal for notifying the operator that there is a possibility that the maintenance information has not been input by the operator and that there is the possibility of the abnormality of the fluid property sensor 10.

Specifically, when an increase or decrease tendency of the detected value from the fluid property sensor 10 is changed (changed so as to have an extremal value), the abnormality detection unit 34 outputs the warning signal to the operator if the maintenance information is not input within a predetermined time period before and after this change. In this embodiment, when the dielectric constant or the conductivity of the working fluid detected by the fluid property sensor 10 is changed from an increase to a decrease, the abnormality detection unit 34 outputs the warning signal to the terminal 20 via the processing-unit-side transmission unit 35 if the maintenance information is not input within the predetermined time period before and after this change. Here, the phrase "changed from an increase to a decrease" refers to a change in the dielectric constant or the conductivity by a predetermined amount or more, and it does not include minor changes caused by noise. It is possible to detect the extremal value of the detected value from the fluid property sensor 10 by removing the noise using filter processing and by determining a point at which the slope of the detected value from the fluid property sensor 10 is changed from positive to negative. In addition, "a predetermined time period" is set to a duration that allows the operator to input, to the input unit 21, the maintenance information indicating that the operator has performed the maintenance work before and after the maintenance of the fluid pressure apparatus 1. For example, "the predetermined time period" is set to a duration of about half a day. As a result, the operator can notice that the maintenance information has not been input, and in a case in which the maintenance has not been performed or the maintenance information has already been input, the operator can notice that there is the abnormality of the fluid property sensor 10.

Furthermore, immediately after the start of operation of the fluid pressure apparatus 1, because the temperature of the working fluid is increased, the electric characteristic value is changed regardless of the degree of degradation of the working fluid. Thus, the abnormality detection unit 34 does not notify the operator of the abnormality of the fluid property sensor 10 and does not output the warning signal within a predetermined operation time from the start of the operation of the fluid pressure apparatus 1. Specifically, the "predetermined operation time" is a time period since the start of the operation of the fluid pressure apparatus 1 until the temperature of the working fluid detected by the fluid property sensor 10 becomes a predetermined value or higher. In addition, "a predetermined value" of the temperature of the working fluid is set set to be close to and smaller than a value at which the temperature of the working fluid is stabilized after the start of the operation of the fluid pressure apparatus 1. For example, in winter, the temperature of the working fluid is about 10 degrees when the fluid pressure apparatus 1 is not being operated, and about 40 to 60 degrees when the fluid pressure apparatus 1 is being operated. In this case, "a predetermined value" of the temperature of the working fluid is set to a temperature between 10 degrees and 40 degrees. As a result, immediately after the start of the operation of the fluid pressure apparatus 1 when the detected value from the fluid property sensor 10 is unstable, it is possible to prevent erroneous notification to the operator regarding the abnormality of the fluid property sensor 10 and the change in the increase or decrease tendency of the detected value.

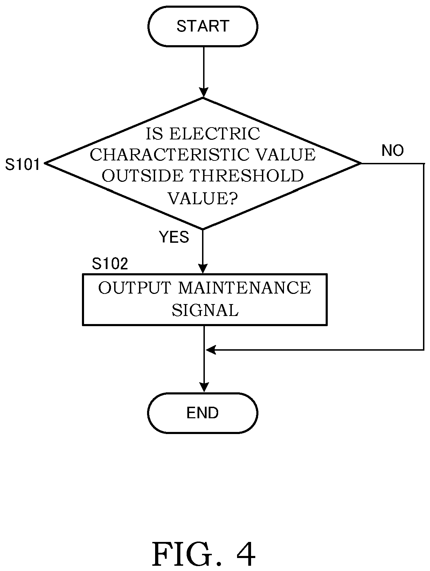

FIG. 4 is a flowchart summarizing the above-described processing of outputting the maintenance signal by the information processing unit 30 to encourage the maintenance of the fluid pressure apparatus 1. The information processing unit 30 repeatedly executes the processing shown in FIG. 4 every time the detected value is input from the fluid property sensor 10 via the processing-unit-side receiver unit 31.

As shown in FIG. 4, when the detected value is input from the fluid property sensor 10, in Step S101, the information processing unit 30 determines whether or not the electric characteristic value of the fluid property sensor 10 (specifically, the dielectric constant and the conductivity) falls within the predetermined threshold value. In Step S 101, if it is determined that the electric characteristic value of the fluid property sensor 10 falls within the predetermined threshold value (i.e., within the threshold value), the processing is terminated without outputting the maintenance signal. In Step S101, if it is determined that the electric characteristic value of the fluid property sensor 10 does not fall within the predetermined threshold value (i.e., outside the threshold value), the processing proceeds to Step S102 to output the maintenance signal to the terminal 20 via the processing-unit-side transmission unit 35, and then, the processing is terminated. In the terminal 20, when the maintenance signal is input, the display unit 24 displays the information encouraging the maintenance of the fluid pressure apparatus 1, thereby notifying the operator.

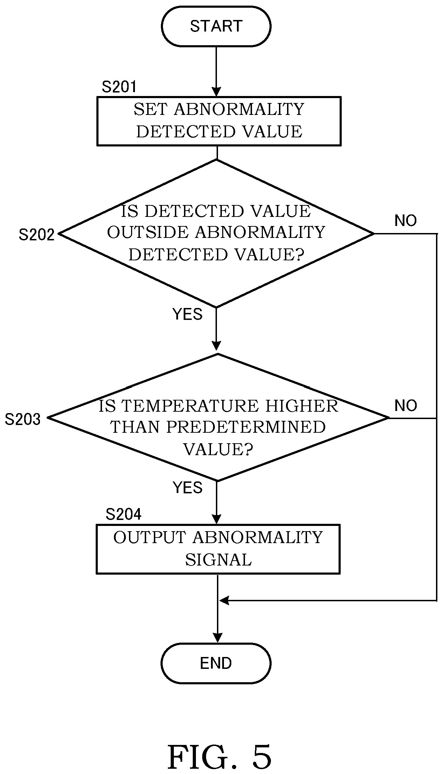

FIG. 5 is a flowchart summarizing the above-described processing of outputting the abnormality signal notifying the abnormality of the fluid property sensor 10 by the information processing unit 30. The information processing unit 30 repeatedly executes the processing shown in FIG. 5 every time the detected value is input from the fluid property sensor 10 via the processing-unit-side receiver unit 31.

As shown in FIG. 5, first of all, in Step S201, the information processing unit 30 sets the abnormality detection value by estimating the detected value from the fluid property sensor 10 from the maintenance information that has been input. Next, the processing proceeds to Step S202, and it is determined whether or not the detected value from the fluid property sensor 10 falls within he abnormality detection value. In Step S202, if it is determined that the detected value from the fluid property sensor 10 falls within the abnormality detection value (i.e., within the abnormality detection value), the processing is terminated without outputting the abnormality signal. In Step S202, if it is determined that the detected value from the fluid property sensor 10 does not fall within the abnormality detection value (i.e., outside the abnormality detection value), the processing proceeds to Step S203, and it is determined whether or not the temperature of the working fluid detected by the fluid property sensor 10 is higher than the predetermined value (in other words, it is determined whether or not it is within a predetermined time period since the start of the operation of the fluid pressure apparatus 1). In Step S203, if it is determined that the temperature of the working fluid detected by the fluid property sensor 10 is lower than the predetermined value (it is within the predetermined time period since the start of the operation of the fluid pressure apparatus 1), the processing is terminated without outputting the abnormality signal. In Step S203, if it is determined that the temperature of the working fluid detected by the fluid property sensor 10 is higher than the predetermined value, the processing proceeds to Step S204 to output the abnormality signal to the terminal 20 via the processing-unit-side transmission unit 35, and then, the processing is terminated. In the terminal 20, when the abnormality signal is input, the display unit 24 displays the information encouraging the maintenance of the fluid property sensor 10, thereby notifying the operator. In addition, if both of the maintenance signal and the abnormality signal are input, the terminal 20 gives priority to the notification by the abnormality signal over the notification by the maintenance signal. The terminal 20 may perform both of the notification by the maintenance signal and the notification by the abnormality signal in combination. In addition, the order is not limited to the above, and Step S203 may be executed before Step S201 or Step S202.

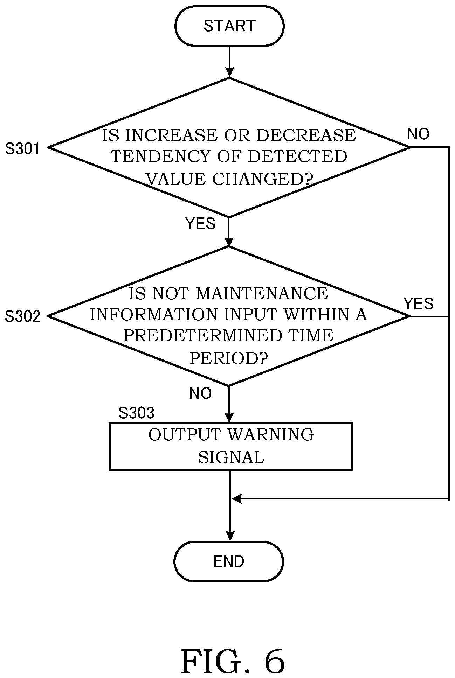

FIG. 6 is a flowchart summarizing the above-described processing of outputting the warning signal by the information processing unit 30. The information processing unit 30 repeatedly executes the processing shown in FIG. 6 every time the abnormality signal is output. In other words, the processing of outputting the warning signal is performed in a state in which the abnormality of the fluid property sensor 10 is detected.

As shown in FIG. 6, first of all, in Step S301, the information processing unit 30 determines whether or not the increase or decrease tendency of the detected value from the fluid property sensor 10 has been changed (specifically, whether or not the dielectric constant or the conductivity has been changed from the increase to the decrease). In Step S301, if it is determined that the increase or decrease tendency of the detected value from the fluid property sensor 10 has not been changed, the processing is terminated without outputting the warning signal. In Step S301, if it is determined that the increase or decrease tendency of the detected value from the fluid property sensor 10 has been changed, the processing proceeds to Step S302, and it is determined whether or not the maintenance information has not been input within the predetermined time period before and after the change. In Step S302, if it is determined that the maintenance information has been input within the predetermined time period, the processing is terminated without outputting the warning signal. In Step S302, if it is determined that the maintenance information has not been input within the predetermined time period, the processing proceeds to Step S303 to output the warning signal to the terminal 20 via the processing-unit-side transmission unit 35, and then, the processing is terminated. In the terminal 20, when the warning signal is input, the display unit 24 displays the information encouraging input of the maintenance information and displays that there is the possibility of the abnormality of the fluid property sensor 10, thereby notifying the operator. As described above, when the warning signal is input in addition to the abnormality signal, the terminal 20 gives priority to the notification by the warning signal over the notification by the abnormality signal. The terminal 20 may perform both of the notification by the abnormality signal and the notification by the warning signal in combination.

As described above, in the fluid property monitoring system 100, the deterioration of the working fluid of the fluid pressure apparatus 1 is detected from the detected value from the fluid property sensor 10, and the notification is provided to the operator to encourage the maintenance of the fluid pressure apparatus 1. In addition, in the fluid property monitoring system 100, the abnormality in the detected value from the fluid property sensor 10 is detected by comparing the abnormality determination value that is set on the basis of the maintenance information input by the operator with the detected value from the fluid property sensor 10, and thereby, the operator is notified of the abnormality. In other words, in the fluid property monitoring system 100, it is possible to determine whether the cause of the detection of the deterioration of the working fluid is due to the actual deterioration of the working fluid or due to the abnormality of the fluid property sensor 10. As a result, it is possible to notify the operator of the abnormality of the fluid property sensor 10 and encourage the operator to perform the maintenance of the fluid property sensor 10, and thus, the decrease in the detection accuracy of the property of the fluid due to the abnormality of the fluid property sensor 10 is prevented.

Furthermore, in the fluid property monitoring system 100, when the increase or decrease tendency of the detected value from the fluid property sensor 10 is changed, if the maintenance information is not input within the predetermined time period before and after the change, the warning signal is output to notify the operator of this situation. Thus, the operator can notice that the maintenance information has not been input, and when the maintenance has not been performed or when the maintenance information has been input, the operator can notice the abnormality of the fluid property sensor 10. In addition, in the fluid property monitoring system 100, the deterioration of the working fluid of the fluid pressure apparatus 1 is detected from an electrical parameter, such as the dielectric constant or the conductivity, rather than a physical parameter, such as a pressure or a viscosity. Therefore, a detection sensitivity for the deterioration of the working fluid is high.

In addition, in the fluid property monitoring system 100, within the predetermined time period from the start of the operation of the fluid pressure apparatus 1, the abnormality of the fluid property sensor 10 is not notified to the operator and the warning signal is not output. As a result, erroneous detection of the abnormality in the detected value from the fluid property sensor 10 and erroneous output of the warning signal indicating that the increase or decrease tendency of the detected value is changed for the operator are prevented immediately after the start of the operation of the fluid pressure apparatus 1 when the detected value from the fluid property sensor 10 is unstable.

In the fluid property monitoring system 100 in this embodiment, the information processing unit 30 outputs the abnormality signal and the warning signal to the terminal 20, and notifies the operator that there is the abnormality of the fluid property sensor 10 and that the increase or decrease tendency of the detected value from the fluid property sensor 10 has been changed without the maintenance information being input. The present invention is not limited thereto, and the information processing unit 30 may output the abnormality signal and the warning signal to a device other than the terminal 20 as long as the operator can be notified of the above matters. In addition, the maintenance information is not limited to that input by the operator to the terminal 20, and it may be possible to employ a configuration in which the maintenance information input by the operator is input to the information processing unit 30. In other words, the terminal 20 is not an essential configuration of the fluid property monitoring system 100.

In addition, in the fluid property monitoring system 100, it is not essential that the information processing unit 30 outputs the warning signal, and it may be possible to employ a configuration in which the information processing unit 30 detects the abnormality of the fluid property sensor 10 and notifies the operator. Furthermore, although there is a possibility of erroneous notification that there is the abnormality of the fluid property sensor 10 and that the increase or decrease tendency of the detected value has been changed, in the fluid property monitoring system 100, it may be possible to employ a configuration in which the information processing unit 30 outputs the abnormality signal and the warning signal immediately after the start of operation of the fluid pressure apparatus 1.

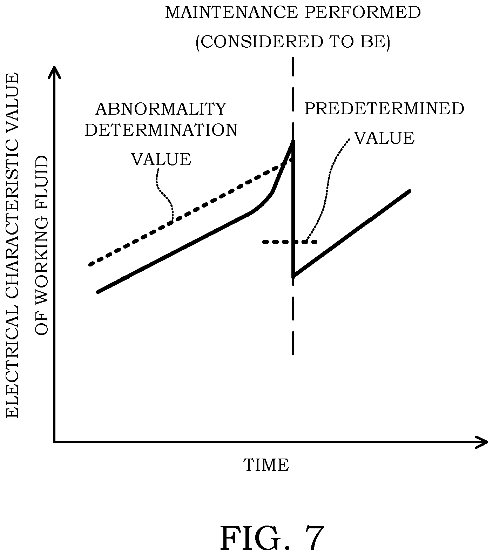

In addition, in the fluid property monitoring system 100, even if the maintenance information is not input by the operator, as shown in FIG. 7, in a case in which the detected value from the fluid property sensor 10 exceeds the abnormality detection value, and thereafter, the detected value becomes equal to or less than predetermined value, it may be possible to consider that the maintenance of the fluid pressure apparatus 1 has been performed. In addition, in a case in which a changed amount of the detected value from the fluid property sensor 10 (the slope of the graph shown in FIG. 7) exceeds the predetermined value, and thereafter, the detected value becomes equal to or less than the predetermined value, it may be possible to consider that the maintenance of the fluid pressure apparatus 1 has been performed. Furthermore, by referring to the detected value that has become equal to or less than the predetermined value, it may be considered that any of the replacement of the working fluid, the addition of the working fluid, and the replacement of the filter has been performed as the maintenance. When it is considered that the maintenance of the fluid pressure apparatus 1 has been performed, the signal indicating this situation is output.

In addition, in the fluid property monitoring system 100, the electric characteristic value to be detected by the fluid property sensor 10 of the working fluid is the dielectric constant or the conductivity, and the dielectric constant or the conductivity is increased as the working fluid is deteriorated due to the increase in the operation time of the fluid pressure apparatus 1. The dielectric constant or the conductivity is decreased when the working fluid is replaced with fresh working fluid that is not deteriorated. The present invention is not limited thereto, and the electric characteristic value to be detected by the fluid property sensor 10 of the working fluid may also be a parameter other than the dielectric constant or the conductivity that is decreased with the deterioration of the working fluid. In this case, the electric characteristic value is increased when the working fluid is replaced with fresh working fluid that is not deteriorated.

In addition, the processing performed by the abnormality determination value setting unit 33 and the abnormality detection unit 34 may be provided as a program for causing a computer to execute the processing. In other words, the program for executing the processing performed by the abnormality determination value setting unit 33 and the abnormality detection unit 34 is a program for causing the computer to execute the detection of the abnormality in the detected value from the fluid property sensor 10 that is attached to the fluid pressure apparatus 1 and detects the property of the fluid, and the program causes the computer to detect the abnormality in the detected value by comparing the detected value from the fluid property sensor 10 with the abnormality determination value that is set on the basis of the maintenance information including the state of the fluid pressure apparatus 1 that is input by the operator.

The program for executing the above-described series of processing is provided in the form of a computer readable memory medium. For example, as the various programs executed by the computer, those stored in, for example, a non-transitory computer readable medium such as a CD-ROM, etc. may also be used. In addition, the various programs executed by the computer may be applications that are provided via a network.

According to the above-described embodiment, following operational advantages are afforded.

In the fluid property monitoring system 100, the information processing unit 30 detects the abnormality in the detected value from the fluid property sensor 10 by comparing the abnormality determination value with the detected value from the fluid property sensor 10 and notifies the operator. Therefore, it is possible to encourage the operator to perform the maintenance of the fluid property sensor 10, and thus, the decrease in the detection accuracy of the property of the fluid due to the abnormality of the fluid property sensor 10 is prevented.

In the fluid property monitoring system 100, when the increase or decrease tendency of the detected value from the fluid property sensor 10 is changed, if the maintenance information is not input within the predetermined time period before and after the change, the information processing unit 30 outputs the warning signal to notify the operator of this situation. Therefore, the operator can notice that the maintenance information has not been input, and when the maintenance has not been performed or when the maintenance information has been input, the operator can notice the abnormality of the fluid property sensor 10.

In the fluid property monitoring system 100, in the predetermined operation time from the start of the operation of the fluid pressure apparatus 1, the information processing unit 30 does not notify the abnormality of the fluid property sensor 10 to the operator and does not output the warning signal, and therefore, immediately after the start of operation of the fluid pressure apparatus 1, it is possible to prevent erroneous notification to the operator regarding the abnormality of the fluid property sensor 10 and the change in the increase or decrease tendency of the detected value.

The configurations, operations, and effects of the embodiment of the present invention configured as described above will be collectively described.

The fluid property monitoring system 100 includes the information processing unit 30 to which the detected value from the fluid property sensor 10 and the maintenance information are input, the fluid property sensor 10 being attached to the fluid pressure apparatus 1 and being configured to detect the property of the fluid, and the maintenance information being input by the operator and including the state of the fluid pressure apparatus 1, wherein the information processing unit 30 is configured to detect the abnormality in the detected value from the fluid property sensor 10 by comparing the detected value with the abnormality determination value set based on the maintenance information and to notify the operator.

With this configuration, the information processing unit 30 detects the abnormality in the detected value from the fluid property sensor 10 by comparing the detected value from the fluid property sensor 10 with the abnormality determination value set on the basis of the maintenance information input by the operator. As a result, it is possible to encourage the operator to perform the maintenance of the fluid property sensor 10, and thus, the decrease in the detection accuracy of the property of the fluid due to the abnormality of the fluid property sensor 10 is prevented.

In addition, when the increase or decrease tendency of the detected value is changed, the information processing unit 30 is configured to output the warning signal if the maintenance information is not input within the predetermined time period before and after this change.

In addition, the detected value includes the dielectric constant or the conductivity of the fluid, and when the dielectric constant or the conductivity of the fluid is changed from the increase to the decrease, the information processing unit 30 is configured to output the warning signal if the maintenance information is not input within the predetermined time period before and after this change.

With these configurations, the operator can notice that the maintenance information has not been input, and when the maintenance has not been performed or when the maintenance information has been input, the operator can notice the abnormality of the fluid property sensor 10.

In addition, the information processing unit 30 is configured not to output the warning signal in the predetermined operation time from the start of the operation of the fluid pressure apparatus 1.

With this configuration, erroneous output of the warning signal indicating that the increase or decrease tendency of the detected value from the fluid property sensor 10 is changed is prevented immediately after the start of the operation of the fluid pressure apparatus 1 when the detected value from the fluid property sensor 10 is unstable.

In addition, the information processing unit 30 is configured not to detect the abnormality in the detected value from the fluid property sensor 10 in the predetermined operation time immediately after the start of operation of the fluid pressure apparatus 1.

In addition, the detected value includes the temperature of the fluid, and the predetermined operation time is a duration from the start of the operation of the fluid pressure apparatus 1 until the temperature of the fluid becomes the predetermined value or higher.

With these configurations, erroneous detection of the abnormality in the detected value from the fluid property sensor 10 is prevented immediately after the start of the operation of the fluid pressure apparatus 1 when the detected value from the fluid property sensor 10 is unstable.

In addition, the method for detecting the abnormality in the detected value from the fluid property sensor 10 that is attached to the fluid pressure apparatus 1 and detects the property of the fluid includes a step of detecting the abnormality in the detected value by comparing the detected value from the fluid property sensor 10 with the abnormality determination value that is set on the basis of the maintenance information including the state of the fluid pressure apparatus 1 that is input by the operator.

With this configuration, the abnormality in the detected value from the fluid property sensor 10 is detected by comparing the detected value from the fluid property sensor 10 with the abnormality determination value set on the basis of the maintenance information input by the operator. As a result, it is possible to encourage the operator to perform the maintenance of the fluid property sensor 10, and thus, the decrease in the detection accuracy of the property of the fluid due to the abnormality of the fluid property sensor 10 is prevented.

In addition, with a program for causing a computer to execute the detection of the abnormality in the detected value from the fluid property sensor 10 that is attached to the fluid pressure apparatus 1 and detects the property of the fluid, the abnormality in the detected value is detected by comparing the detected value from the fluid property sensor 10 with the abnormality determination value that is set on the basis of the maintenance information including the state of the fluid pressure apparatus 1 that is input by the operator.

With this configuration, the abnormality in the detected value from the fluid property sensor 10 is detected by comparing the detected value from the fluid property sensor 10 with the abnormality determination value set on the basis of the maintenance information input by the operator. As a result, it is possible to encourage the operator to perform the maintenance of the fluid property sensor 10, and thus, the decrease in the detection accuracy of the property of the fluid due to the abnormality of the fluid property sensor 10 is prevented.

Embodiments of the present invention were described above, but the above embodiments are merely examples of applications of the present invention, and the technical scope of the present invention is not limited to the specific constitutions of the above embodiments.

With respect to the above description, the contents of application No. 2022-59441, with a filing date of March 31, 2022 in Japan, are incorporated herein by reference.

## Claims

1. A fluid property monitoring system comprising
an information processing unit to which a detected value from a fluid property sensor and maintenance information are input, the fluid property sensor being attached to a fluid pressure apparatus and being configured to detect a property of fluid, and the maintenance information being input by an operator and including a state of the fluid pressure apparatus, wherein
the information processing unit is configured to detect an abnormality in the detected value by comparing the detected value and an abnormality determination value set based on the maintenance information.

2. The fluid property monitoring system according to claim 1, wherein
when an increase or decrease tendency of the detected value is changed, the information processing unit is configured to output a warning signal if the maintenance information is not input within a predetermined time period before and after this change.

3. The fluid property monitoring system according to claim 1, wherein
the detected value includes dielectric constant or conductivity of the fluid,
when dielectric constant or conductivity of the fluid is changed from an increase to a decrease, the information processing unit is configured to output a warning signal if the maintenance information is not input within a predetermined time period before and after this change.

4. The fluid property monitoring system according to claim 2 or 3, wherein
the information processing unit is configured not to output the warning signal within a predetermined operation time from start of operation of the fluid pressure apparatus.

5. The fluid property monitoring system according to claim 1, wherein
the information processing unit is configured not to detect an abnormality in the detected value from the fluid property sensor within a predetermined operation time from start of operation of the fluid pressure apparatus.

6. The fluid property monitoring system according to claim 4, wherein
the detected value includes temperature of the fluid, and
the predetermined operation time is a duration from the start of the operation of the fluid pressure apparatus until the temperature of the fluid becomes a predetermined value or higher.

7. A method for detecting an abnormality in a detected value from a fluid property sensor that is attached to a fluid pressure apparatus and detects a property of a fluid, wherein
the abnormality in the detected value is detected by comparing the detected value from the fluid property sensor with an abnormality determination value, the abnormality determination value being set based on maintenance information including a state of the fluid pressure apparatus, and the maintenance information being input by an operator.

8. A program for causing a computer to execute detection of an abnormality in a detected value from a fluid property sensor that is attached to fluid pressure apparatus and detects a property of a fluid, wherein
the abnormality in the detected value is detected by comparing the detected value from the fluid property sensor with an abnormality determination value, the abnormality determination value being set based on maintenance information including a state of the fluid pressure apparatus, and the maintenance information being input by an operator.
